# EUROPÄISCHE PATENTANMELDUNG

(11) **EP 1 987 762 A1**
(43) Veröffentlichungstag der Anmeldung: **05.11.2008**
(21) Anmeldenummer: 07450085.1
(22) Anmeldetag: 03.05.2007
(51) Int. Cl.: A61B 5/00, G01N 33/49

(54) **Oximeter**

(71) Anmelder: F.HOFFMANN-LA ROCHE AG, 4070 Basel (CH); Roche Diagnostics GmbH, 68305 Mannheim (DE)
(72) Erfinder: Stimpfl, Peter, A-8053 Graz-Neuhart (AT); Huemer, Herfried, Dipl.-Ing., A-8330 Feldbach (AT); Strohmeier, Manfred, Ing., A-8010 Graz (AT); Untersberger, Stefan, A-8045 Graz (AT)
(74) Vertreter: Babeluk, Michael

(57) **Zusammenfassung**

Ein Oximeter zur spektralphotometrischen in vitro-Bestimmung von Hämoglobinderivaten in einer Probe, vorzugsweise einer hämolysierten Blutprobe, mit einer einzigen Messlichtquelle (1), welche eine Messstrahlung emittiert, mit einer Probenkammer (5), beispielsweise einer Messküvette zur Aufnahme der Probe und einer Detektionseinrichtung (9,10). Die Messlichtquelle (1) ist eine polychromatische LED, welche zur Bestimmung der Hämoglobinderivate Messstrahlung zumindest in einem Spektralbereich B emittiert, in welchem die Hämoglobinderivate eine signifikante Absorption aufweisen und zur Erfassung mindestens eines weiteren Analyten Messstrahlung zumindest in einem weiteren Spektralbereich A emittiert, in welchem der mindestens eine weitere Analyt eine signifikante Absorption aufweist.

## Beschreibung

Die Erfindung betrifft ein Oximeter zur spektralphotometrischen in vitro-Bestimmung von Hämoglobinderivaten in einer Probe, vorzugsweise einer hämolysierten Blutprobe, mit einer einzigen Messlichtquelle, welche eine Messstrahlung emittiert, mit einer Probenkammer, beispielsweise einer Messküvette zur Aufnahme der Probe und einer Detektionseinrichtung.

In diesem Zusammenhang ist aus der JP 2004-108781 ein Spektroskop bekannt geworden, in welchem das von einer Weißlicht-LED emittierte Licht bereits vor dem Einstrahlen in eine Probe mittels zweier Beugungsgitter in seine spektralen Bestandteile aufgespaltet und über einen Projektionsschlitz in die Probe eingestrahlt wird, um beispielsweise einen Analyten in der Probe zu bestimmen. Es sind sowohl eine Varianten mit einer Transmissionsgeometrie als auch Varianten mit einer Reflexionsgeometrie beschrieben, wobei die jeweils transmittierte bzw. reflektierte Lichtintensität nacheinander für jede Wellenlänge gemessen wird. Nachteilig bei diesem bekannten Spektroskop ist die zeitaufwendige Erfassung eines Spektrums mit Hilfe der beiden Beugungsgitter, welche das Messlicht in seine spektralen Bestandteile aufspalten.

Aus der US 2005/0154277 A1 ist ein miniaturisiertes "in vivo"-Spektroskop bekannt, welches innerhalb des Körpers beispielsweise Blutungen im Gastrointestinaltrakt mittels spektraler Analyse der dort eventuell vorhandenen Hämoglobinderivate ermitteln kann. Als Lichtquelle können unter anderem LEDs verwendet werden.

Weiters ist es aus der US 2005/0267346 A1 bekannt, ein nicht invasives Oximeter zu verwenden, bei welchem in gut durchblutetes Gewebe an der Fingerkuppe oder am Ohrläppchen Licht eingestrahlt wird, wobei auf der Basis der Absorption durch das Blut im Durchlicht- oder Reflexionsverfahren Rückschlüsse auf die Zusammensetzung (Sauerstoffsättigung) des Blutes gemacht werden. Als Lichtquelle kann eine Weißlicht-LED eingesetzt werden, wobei jedoch vor dem Einstrahlen ins Gewebe Filter oder Beugungsgitter verwendet werden müssen, um nur definierte Wellenlängen ins Gewebe einzustrahlen.

Aus der US 6,262.798 B1 ist ein Oximetrieverfahren zur Durchführung von Messungen an nicht hämolysiertem Blut bekannt. Bei diesem Messverfahren wird nacheinander eine Vielzahl definierter, monochromatischer Wellenlängen in die Probe eingestrahlt, wobei Arrays verschiedenfarbiger LEDs oder herkömmliche Weißlichtlampen eingesetzt werden, aus welchen mittels eines Monochromators definierte Wellenlängenbereiche ausgefiltert werden, welche dann in die Probe eingestrahlt werden.

Zur Herstellung eines polychromatischen Lichtes mit LEDs sind sogenannte Lumineszenzkonversions-LEDs bekannt geworden, welche eine oder mehrere primäre Emissionswellenlängen aufweisen, die durch Lumineszenzkonversionsschichten so modifiziert werden, dass letztendlich ein breitbandiges polychromatisches Licht abgestrahlt wird. Derartige Lichtquellen werden beispielsweise in der US 2005/0127358 A1 beschrieben. Das abgestrahlte polychromatische Licht setzt sich hierbei aus einem kurzwelligem Spektralbereich der primären Emissionswellenlängen, welche von einem LED-Chip als Primäremitter abgestrahlt werden und einem langwelligeren Spektralbereich, welcher von den durch die Primäremission der LED angeregten Farbstoffschichten als Sekundäremitter abgestrahlt wird, zusammen.

Weiters beschreibt die US 6,809,347 B2 eine Weißlicht-LED, welche aus einer Blau- bzw. UV-Licht emittierenden LED und einer darüber angebrachten Luminophorschicht besteht, die einen Teil des von der LED imitierten Blau- bzw. UV-Lichts absorbiert und anschließend im langwelligen Spektralbereich Licht emittiert, so dass durch Überlagerung weißes Licht entsteht, dessen spektrale Zusammensetzung durch Modifikation der Luminophorschicht definiert werden kann.

Schließlich beschreibt die EP 1 473 771 A1 eine alternative Konstruktionsform einer Weißlicht-LED, welche aus mehreren zumindest teilweise transparenten lichtemittierenden LED-Schichten unterschiedlicher Emissionswellenlängen besteht, welche übereinander angeordnet sind, so dass in Abstrahlungsrichtung die einzelnen Emissionswellenlängenbereiche überlagert werden und in Summe weißes Licht emittiert wird.

Aufgabe der Erfindung ist es, ein Oximeter zur spektralphotometrischen in vitro-Bestimmung von Hämoglobinderivaten in einer vorzugsweise medizinischen Probe derart weiterzubilden, dass ein kompaktes Messmodul entsteht, mit welchem eine rasche Erfassung der Messspektren möglich ist, wobei neben den Hämoglobinderivaten weitere Analyte erfasst werden sollen. Weiters soll eine ausreichende Stabilität (geringes Driften) und eine lange Lebensdauer der Lichtquelle gewährleistet sein. Das Oximeter soll eine höhere Benutzerfreundlichkeit, weniger Wartungsaufwand aufgrund der längeren Standzeit der Lichtquelle und eine hohe Genauigkeit der Messergebnisse aufweisen.

Diese Aufgabe wird erfindungsgemäß dadurch gelöst, dass die Messlichtquelle eine polychromatische LED ist, welche zur Bestimmung der Hämoglobinderivate Messstrahlung zumindest in einem Spektralbereich B emittiert, in welchem die Hämoglobinderivate eine signifikante Absorption aufweisen und zur Erfassung mindestens eines weiteren Analyten Messstrahlung zumindest in einem weiteren Spektralbereich A emittiert, in welchem der mindestens eine weitere Analyt eine signifikante Absorption aufweist. Die Messstrahlung muss in einem derart signifikanten Ausmaß absorbiert werden, dass beispielsweise für ein Mehrkomponentenanalyseverfahren hinreichend differenzierbare Absorptionswerte der Hämoglobinderivate und des weiteren Analyten vorliegen. Solche geeigneten Spektralbereiche sind beispielsweise für die Bestimmung von Hämoglobinderivaten der Bereich von 520 - 670 nm und für die Bestimmung von Bilirubin der Bereich von 450 - 500 nm.

Unter dem Begriff polychromatische LED wird im Wesentlichen eine Weißlicht-LED z.B. gemäß eingangs zitierter US 2005/0127358 A1, US 6,809,347 B2 oder EP 1 473 771 A1 verstanden, deren Emissionswellenlängenbereich und Intensitätsverlauf an die Absorptionseigenschaften der zu bestimmenden Hämoglobinderivate angepasst und gegebenenfalls erweitert wird, um einen zusätzlichen Analyten zu bestimmen.

Gemäß einer ersten Variante der Erfindung kann die Messlichtquelle eine Lumineszenzkonversions-LED sein, welche mindestens einen Primäremitter und mindestens einen Sekundäremitter beinhaltet, wobei der Primäremitter Messstrahlung im Spektralbereich A und der Sekundäremitter im Spektralbereich B emittiert.

Bei einer besonders bevorzugten Lösung, basierend auf einer Lumineszenzkonversions-LED, wird der Spektralverlauf (Lichtintensität in Abhängigkeit der Wellenlänge) durch Art, Anzahl und Menge der verwendeten Phosphore, sowie durch geeignete Wahl der Anregungswellenlänge (Art und Zahl der Primäremitter) eingestellt und optimal auf die zu bestimmenden Analyten der Probe abgestimmt. Diese Bauform einer Messlichtquelle hat gegenüber konventionellen Lichtquellen den weiteren Vorteil, dass das emittierte Licht über die zur Messung genutzte Austrittsfläche der Messlichtquelle bzw. über den zur Messung genutzten Abstrahlwinkelbereich der Messlichtquelle ein im Wesentlichen homogenes Spektrum aufweist. Solche Emissionseigenschaften sind insbesondere für die Reduktion der Toleranzempfindlichkeit in Bezug auf die Positionierung der optischen Komponenten des Messsystems des Oximeters von Bedeutung. Hierbei ist insbesondere die Größe der abstrahlenden Fläche einer LED im Vergleich zu der einer konventionellen Halogenlampe vorteilhaft, welche eine möglichst fehlertolerante Ausrichtung des optischen Systems des Oximeters ermöglicht.

Gemäß einer zweiten Variante der Erfindung beinhaltet die Messlichtquelle mehrere lichtemittierende Schichten mit unterschiedlichen Emissionsspektren, wobei zumindest eine der lichtemittierenden Schichten Messstrahlung im Spektralbereich A und zumindest eine weitere der lichtemittierenden Schichten Messstrahlung in einem Spektralbereich B emittiert, wobei die lichtemittierenden Schichten innerhalb der Messlichtquelle so zueinander angeordnet sind, dass die Messstrahlung über die zur Messung genutzte Austrittsfläche der Messlichtquelle bzw. über den zur Messung genutzten Abstrahlwinkelbereich der Messlichtquelle ein im Wesentlichen homogenes Spektrum aufweist.

Gemäß dieser Ausführungsform können auch einzelne lichtemittierende Schichten oder Einzelemitter (SMD oder konventionelle LEDs) zur Erzeugung der Messstrahlung verwendet werden, sofern diese in hinreichender enger räumlicher Nähe angeordnet sind, so dass insbesondere die zur Messung genutzte Messstrahlung ein im Wesentlichen homogenes Spektrum über die Austrittsfläche der Messlichtquelle aufweist. Der Spektralverlauf kann durch Art, Anzahl sowie der Betriebsparameter der einzelnen emittierenden Schichten oder Einzelemitter eingestellt werden. Unter dem Begriff hinreichend enge räumliche Nähe ist zur verstehen, dass, wenn diese Lichtquelle durch die Probe in eine Detektionseinheit eingestrahlt wird, die Positionierung der optischen Komponenten unempfindlich gegenüber geometrischen Toleranzen ist. Demnach verändert sich sowohl Intensität als auch Spektrum des detektierten Lichtes auch bei Abweichungen von der idealen Positionierung (z. B. Sollposition der optischen Komponenten entlang der optischen Achse) der optisch relevanten Komponenten nur unwesentlich. Vorteilhafte Auswirkung dessen ist ein gegen Dejustierungen deutlich robusteres und weniger anfälliges optisches System.

Die erfindungsgemäße Lösung besteht somit im Einsatz einer polychromatischen LED zur spektrometrischen Bestimmung aller Hämoglobinderivate und mindestens einer weiteren Substanz. Solche weiteren Substanzen weisen hierbei auch eine Lichtabsorption außerhalb des Absorptionsbereichs der Hämoglobinderivate auf, welche zu deren spektrometrischen Bestimmung genutzt werden kann.

Ein Beispiel eines derartigen Analyten ist Bilirubin, welches beispielsweise anhand seiner Absorption im Wellenlängenbereich von 450-500 nm (entspricht Spektralbereich A der Messstrahlung) bestimmt werden kann.

Ein geeigneter Spektralbereich zur Bestimmung der Hämoglobinderivate ist insbesondere der Wellenlängenbereich von 520 - 670 nm (entspricht Spektralbereich B der Messstrahlung).

Gemäß einer Variante weist die Messlichtquelle mehrere lichtemittierende Schichten mit unterschiedlichen Emissionsspektren auf, welche Schichten zumindest teilweise transparent sind und stapelartig übereinander angeordnet sind, so dass durch die Überlagerung der Emissionsstrahlung der einzelnen lichtemittierenden Schichten in Emissionsrichtung eine Gesamtstrahlung entsteht, die über die Austrittsfläche der Messlichtquelle eine im Wesentlichen homogene Spektralverteilung aufweist.

Gemäß einer weiteren Variante kann die Messlichtquelle mehrere lichtemittierende Bereiche oder Einzelemitter mit unterschiedlichen Emissionsspektren enthalten, welche in einem so nahen Abstand nebeneinander angeordnet sind, so dass durch die Überlagerung der Emissionsstrahlung der einzelnen lichtemittierenden Bereiche oder Einzelemitter in Emissionsrichtung eine Gesamtstrahlung entsteht, die über die Austrittsfläche der Messlichtquelle eine im Wesentlichen homogene Spektralverteilung aufweist.

Das Emissionsspektrum einer solchen Messlichtquelle stellt ein Summenspektrum dar und setzt sich additiv aus den einzelnen Emissionsspektren der jeweiligen lichtemittierenden Schichten zusammen, wobei je nach Positionierung der einzelnen lichtemittierenden Schichten und/oder dem Vorhandensein lichtschwächender Schichten innerhalb der Messlichtquelle die jeweiligen Emissionspektren der einzelnen lichtemittierenden Schichten vor dem Austritt aus der Messlichtquelle noch modifiziert werden können.

Das von der Messlichtquelle emittierte Licht sollte über die zur Messung genutzte Austrittsfläche der Messlichtquelle bzw. über den zur Messung genutzten Abstrahlwinkelbereich der Messlichtquelle ein im Wesentlichen homogenes Spektrum aufweisen. Unter der zur Messung genutzten Austrittsfläche der Messlichtquelle bzw. dem zur Messung genutzten Abstrahlwinkelbereich der Messlichtquelle ist insbesondere der Bereich der Austrittsfläche der Messlichtquelle zu verstehen, welcher durch das optische System des Oximeters in die Probe eingestrahlt und letztendlich auf die Detektionseinrichtung abgebildet wird. Insbesondere über diesen Bereich sollte die emittierte Messstrahlung eine möglichst homogene spektrale Zusammensetzung aufweisen. Unter homogener spektraler Zusammensetzung über eine Fläche wird hierbei verstanden, dass an allen Orten innerhalb dieser Fläche Licht emittiert wird, welches unabhängig vom genauen Emissionsort stets eine gleiche spektrale Zusammensetzung aufweist (gleiche Emissionswellenlängenbereiche und gleiche Intensität der jeweils emittierten Strahlung).

Die geometrische Emissionscharakteristik des Messlichtquelle kann vorteilhafterweise noch zusätzlich durch lichtstreuende Diffusorschichten zur Homogenisierung des abgestrahlten Messlichtes modifiziert werden. Solche Diffusorelemente können sowohl innerhalb der Messlichtquelle als integrale Bestandteile dieser als auch außerhalb der Messlichtquelle als separate optische Elemente im optischen Pfad angeordnet werden.

Die erfindungsgemäße Ausgestaltung der Messlichtquelle bietet eine vorteilhafte Voraussetzung für eine spektrale Anpassung der Messstrahlung in Bezug auf die Optimierung des Signal/Rausch-Verhältnisses über den gesamten Spektralbereich unter Rücksichtnahme auf die Analytabsorption, sowie des wellenlängenabhängigen Falschlichtes in der Detektionseinheit durch gezielte Wahl von Art, Anzahl und Menge von Primäremittern und Sekundäremittern.

Weiterhin kann das Oximeter weitere optische Komponenten wie Filter, Lichtleiter, Linsen, Strahlteiler, Diffusorelemente etc. für die Weiterleitung und Lenkung der Messstrahlung von der Messlichtquelle zur Probenkammer und/oder von der Probenkammer zur Detektionseinrichtung aufweisen.

Insbesondere ist vorgesehen, dass die Detektionseinrichtung aus einem Polychromator und einer nachgeschalteten Multi-Channel-Detektionseinheit, beispielsweise einem alle Messwellenlängen simultan erfassenden Detektorarray, besteht. Dadurch ergeben sich große Vorteile im Vergleich zu einer sequenziellen Messung, wie beispielsweise in der eingangs zitierten JP 2004-108781, wobei vor allem die wesentlich kürzere Messzeit und die Vermeidung mechanisch beweglicher Bauteile (Beugungsgitter etc.) hervorzuheben ist.

Zusätzlich können Filter oder sonstige optisch absorbierende Medien zur weiteren spektralen Anpassung des Spektrums zwischen der Messlichtquelle und der Probenkammer und/oder zwischen der Probenkammer und der Detektionseinrichtung verwendet werden. Beispielsweise können hiermit zur Vermeidung von Falschlicht in der Detektionseinheit und zur Minimierung der Wärmeentwicklung in der Probe und/oder dem optischen System des Oximeters bestimmte analytisch weniger relevante Wellenlängebereiche des von der Messlichtquelle emittierten Lichts zumindest teilweise herausgefiltert werden. Solche Elemente können sowohl als integraler Bestandteil der Messlichtquelle oder als separates, der Messlichtquelle nachgeschaltetes optisches Bauteil ausgebildet sein.

Vorteilhafterweise können so die zur Bestimmung von Hämoglobin und des zumindest einen weiteren Analyten verwendeten Spektralbereiche der Messstrahlung durch einen Bereich geringerer Intensität getrennt sein, insbesondere dann, wenn der analytisch relevante Informationsgehalt der Strahlung in diesem Zwischenbereich klein ist.

Unter Oximeter im Sinne der vorliegenden Anmeldung wird allgemein ein Spektrometer verstanden, mit welchem zumindest die unterschiedlichen Hämoglobinderivate, insbesondere die Hämoglobinderivate Oxyhämoglobin (O2Hb), Desoxyhämoglobin (HHb), Carboxyhämoglobin (COHb) und Methämoglobin (MetHb), aufgrund ihrer unterschiedlichen Absorptionseigenschaften bestimmt werden können.

Übersicht der Vorteile der erfindungsgemäßen polychromatischen LEDs gegenüber konventionellen Glühlampen:
- bessere Stabilität (geringeres Driften);
- kleinere Bauweise;
- geringere Abwärme;
- keine Intensität im IR (folglich kein IR Filter notwendig);
- Lebensdauer im Bereich von 100.000 Stunden möglich (Halogenlampen typ. 5000 Stunden);
- erhöhter Wirkungsgrad gegenüber Glühlampen;
- erhöhte Lichtausbeute;
- Dauerbetriebsoption aufgrund der hohen Lebensdauer;
- Intensität ist über den Strom in einem großen Bereich einstellbar;
- keine mechanische Lichtquellenjustage erforderlich;
- Intensitätsregelung über die Chiptemperatur (Temperaturfühler oder berührungslos mit IR Sensor);
- spektrale Anpassungsmöglichkeit über den Betriebsstrom und den Abstrahlwinkel;
- anwendungsspezifisches Spektrum möglich.

Übersicht der Vorteile der erfindungsgemäßen polychromatischen LEDs gegenüber der Verwendung konventioneller LEDs (bzw. Laserdioden):
- kann wie eine Glühlampe als Lichtquelle mit homogener Spektralverteilung über die gesamte Austrittsfläche betrachtet werden. Damit kann die Abbildungsoptik einfach und kostengünstig gestaltet werden;
- geringe Toleranzempfindlichkeit;
- verbesserte spektrale Anpassungsmöglichkeit möglich;
- kompakteres, einfacheres Temperaturmanagement;
- simultane Erfassung zusätzlicher Analyten, beispielsweise Bilirubin, gemeinsam mit den Hämoglobinparametern möglich.

Das erfindungsgemäße Oximeter eignet sich vor allem für medizinische Proben, welche Blutbestandteile enthalten sowie ggf. einen weiteren Analyten, wie beispielsweise Bilirubin. Es sind dies vor allem Blutproben, insbesondere hämolysiertes Vollblut.

Die erfindungsgemäßen Varianten der Gestaltung einer Messlichtquelle können auch beliebig miteinander kombiniert werden. So können beispielsweise die Emissionspektren einzelner lichtemittierender Schichten einzeln oder auch gemeinsam mit geeigneten Luminophorenschichten überschichtet werden, welche das primäre Emissionsspektrum der einzelnen lichtemittierenden Schichten in den langwelligeren Bereich ausdehnen können. Weiterhin können auch beispielsweise Lumineszenzkonversions-LEDs weitere lichtemittierende Schichten oder auch Einzelemitter hinzugefügt werden, um den Spektralbereich des Emissionslicht auszuweiten und somit beispielsweise zusätzliche Analyten bestimmen zu können.

Die Erfindung wird im Folgenden anhand von Zeichnungen und Diagrammen näher erläutert. Es zeigen:
- Fig. 1: ein erfindungsgemäßes Oximeter in einer schematischen Darstellung,
- Fig. 2: die Extinktionskoeffizienten der Hämoglobinderivate O2Hb, HHb, COHb und MetHb sowie Bilirubin in Abhängigkeit von der Wellenlänge in nm und die
- Fig. 3 bis 6: die Emissionsspektren unterschiedlicher polychromatischer LEDs.

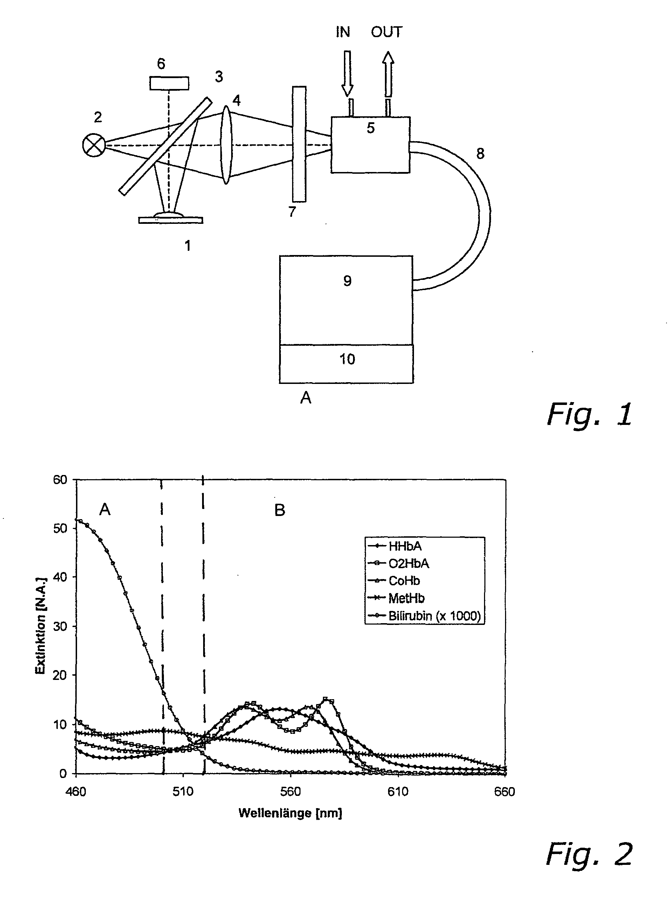

Fig. 1 zeigt in einer schematischen Darstellung den beispielhaften Aufbau eines erfindungsgemäßen Oximeters. Im Oximeter wird eine polychromatische LED 1 als Messlichtquelle zusammen mit einer weiteren Lichtquelle 2 über einen Strahlteiler 3 und ein Linsensystem 4 direkt in die Probenkammer 5, beispielsweise eine Messküvette mit der zu analysierenden Blutprobe, eingekoppelt. In diesem Beispiel wird die weitere Lichtquelle 2 zur Wellenlängenkalibration des Oximeters verwendet.

Die polychromatische LED 1, welche die Messstrahlung in einem Spektralbereich von 450 bis 670 nm breitbandig in die Probenkammer 5 einstrahlt, wird thermisch mittels eines Peltierelementes und eines NTC -Temperaturfühlers thermostatisiert. Die polychromatische LED wird weiters über einen Regelkreis mit einer Photodiode 6 intensitätsgeregelt. Das optische System ist so ausgelegt, dass eine Justage der polychromatischen LED 1 relativ zur optischen Achse aufgrund der großen Austrittsfläche der Messlichtquelle sowie des im Wesentlichen homogenen Abstrahlspektrums über diese Austrittsfläche nicht mehr zwingend erforderlich ist. Optional kann zur Anpassung der Messstrahlung im gewählten Spektralbereich ein Filter 7 zwischen der Messlichtquelle 1 bzw. dem Strahlteiler 3 und der Probenkammer 5 eingesetzt sein. Die Detektionseinrichtung, welche über einen Lichtleiter 8 mit der Messküvette 5 verbunden ist, besteht aus einem Polychromator 9, z.B. einem Gitterspektrometer und einer nachgeschalteten Multi-Channel-Detektionseinheit 10, beispielsweise einem alle Messwellenlängen simultan erfassenden Detektorarray.

Die spektralen Anforderungen an die Messlichtquelle 1 werden in dieser Anwendung vom Spektrometer bzw. von den zu erfassenden Analyten (Hämoglobinderivate und zumindest ein weiterer Analyt) abgeleitet. Der Erfassung der Hämoglobinderivate sowie Bilirubin als exemplarischen weiteren Analyten liegen die Fig. 2 abgebildeten Extinktionskoeffizienten zu Grunde. Zur spektrometrischen Bestimmung der Analyte ist daher eine Messlichtquelle 1 mit entsprechenden Intensitätswerten innerhalb dieser Wellenlängen-Bereiche erforderlich. In spektraler Hinsicht soll die Messlichtquelle 1 der Anwendung entsprechend folgende Eigenschaften aufweisen:
- Abfallende Flanke im langwelligen Bereich zur Reduktion von störendem Falschlicht im Detektor
- Ein Intensitätsmaximum im Spektralbereich B des Absorptionsmaximums der Hämoglobinderivate (520- 670 nm).
- Eine Intensitätsmaximum bzw. eine zur Messung ausreichende (signifikante) Intensität im kurzwelligen Spektralbereich A (450 - 500 nm) zur Erfassung von Bilirubin.
- Eine Intensitätsverringerung zwischen diesen beiden Spektralbereichen A und B.

### Beispiele:

Den im Folgenden angeführten Beispielen liegt ein Oximeter gemäß Fig. 1 zugrunde. Hierzu wurde ein Gitterspektrometer 9 mit einer Wellenlängenauflösung von 1,5 nm und einem Zeilensensor 10 mit einer Pixelanzahl von 512 Pixeln verwendet. Die Messküvette 5 bildet einen fluidischen Kanal mit einer Breite von 1 mm und einer Schichtdicke von 100µm. Die Spektralmessung der polychromatischen LED 1 wurde an einer luftgefüllten Messküvette durchgeführt. Die Einkopplung der Messstrahlung der LED in die Messküvette 5 und in weiterer Folge in den Lichtleiter 8 erfolgt über eine Linse 7 (Vergrößerungsfaktor ca. 1,6). Der Lichtleiter 8 ist als Faserbündel ausgeführt um einen geringen Krümmungsradius des Lichtleiters zwischen Messküvette und Spektrometer herstellen zu können. Der aktive Lichtleiterdurchmesser am Eintritt beträgt ca. 0,7 mm. Am Lichtaustritt werden die Einzelfasern als Faserzeile ausgeführt und bildet somit gleichzeitig den Eintrittsspalt des Spektrometers. Die Photodiode 6 wird zur Regelung der Intensität der LED 1 verwendet. In diesem Beispiel wird die LED 1 zusammen mit einer weiteren Lichtquelle 2 (Neon Glühlampe) mittels eines Strahlteilers 3 kombiniert, um zwischen zwei verschiedenen Beleuchtungsarten (Messstrahlung und Kalibrationsstrahlung) wechseln zu können. Optional können im Aufbau - wie bereits erwähnt - Filterelemente 7 zur weiteren spektralen Anpassung der Messlichtquelle 1 eingefügt werden.

### Beispiel 1:

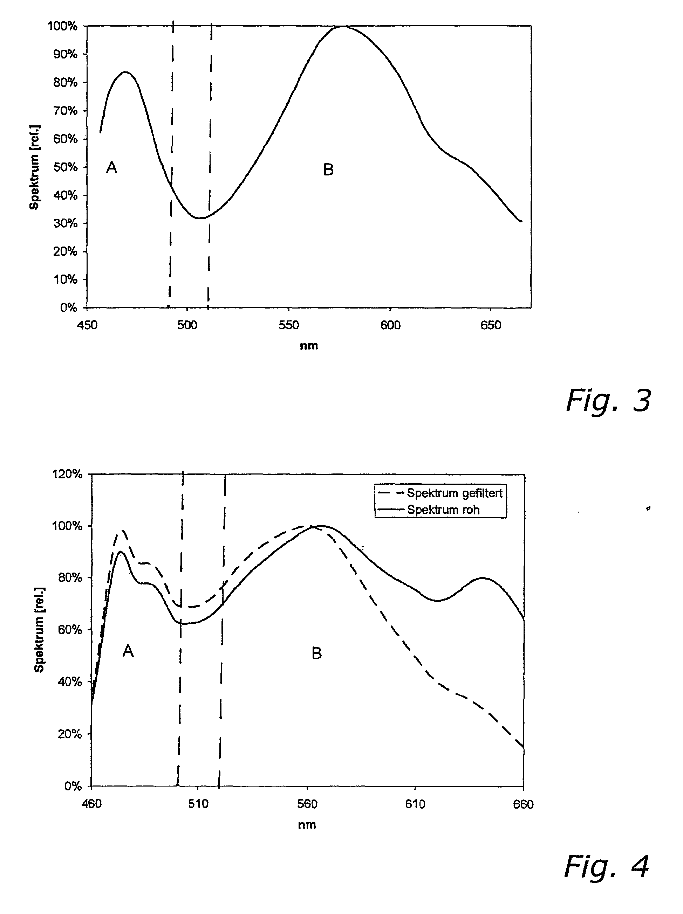

Auf der Basis des oben dargestellten Messaufbaus wurde eine handelsübliche Weißlicht-LED der Firma Seoul Semiconductor (Type: N32180 400mA 3,5 V) verwendet. Das breitbandige Spektrum (siehe Fig. 3) erlaubt die messtechnische Erfassung der Hämoglobinderivate und Bilirubin. Das Verhältnis zwischen Anregungsintensität und Lumineszenzintensität sowie der Intensitätsabfall über 600 nm sind der Anwendung entsprechend geeignet.

### Beispiel 2

In diesem Beispiel wurde eine Weißlicht LED der Firma Luxeon (Type: LXHL 1 Watt) eingesetzt (siehe Spektrum gemäß Fig. 4). Diese LED erlaubt ebenfalls die messtechnische Erfassung der Hämoglobinderivate und Bilirubin, erfordert allerdings zur Verringerung des Falschlichtes im Spektrometer die Verwendung von zusätzlichen Filtern im langwelligen Bereich. In Fig. 4 ist das Spektrum ungefiltert sowie mit einem Filter (BG38 von Schott, 2 mm) dargestellt. Diese Lichtquelle ist insbesondere in Kombination mit einem derartigen Filter somit der Anwendung entsprechend geeignet.

### Beispiel 3

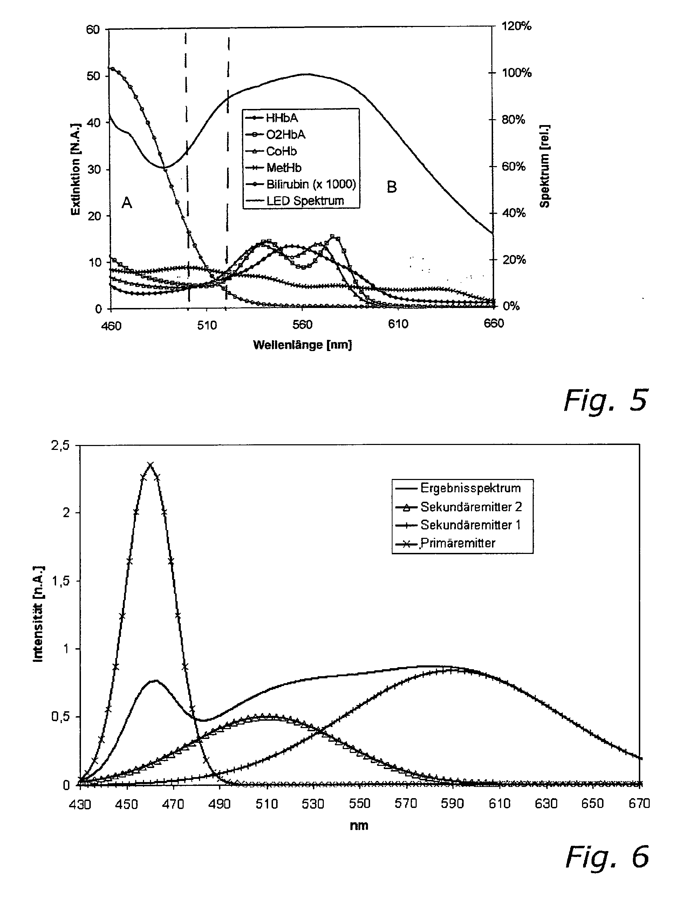

Als bevorzugte Variante kommt eine polychromatische LED mit zwei Phosphoren und einer Farbtemperatur von 4000°K zum Einsatz. Das benötigte Spektrum wird durch einen LED-Chip der dominanten Wellenlänge von 460-462,5 nm, kombiniert mit zwei Luminophoren (Phosphor 1: Grün, CIE Koordinaten: x = 0,195 +/-0,004 y = 0,651 +/- 0,004 und Phosphor 2: Orange, CIE Koordinaten: x = 0,450 +/- 0,002 y = 0,537 +/- 0,002), erreicht. Diese LED ist auf einen Betriebsstrom von 350 mA ausgelegt. In diesem Beispiel beträgt der Betriebsstrom 100 mA. Das gewonnene Spektrum ist in Fig. 5 mit den Extinktionskoeffizienten der Analyten dargestellt. Diese Lichtquelle erfüllt alle Anforderungen die an die Messlichtquelle (1) gestellt werden. Eine zusätzliche Anpassung des Spektrums mit Filtern ist hier nicht erforderlich.

Fig. 6 zeigt das Ergebnisspektrum einer solchen Lumineszenzkonversions-LED anhand eines Beispieles mit zwei Luminophoren (510 nm und 590 nm) als Sekundäremitter und einem Primäremitter der bei 460 nm emittiert. Hier werden blau oder auch UV emittierende LED-Chips mit Lumineszenzfarbstoffen kombiniert. Diese Farbstoffe sind in einer Paste enthalten, die auf den LED-Chip aufgebracht wird. Das kurzwellige und damit energiereichere blaue Licht regt den bzw. die Farbstoffe zum Leuchten an. Dabei wird langwelligeres, energieärmeres Licht abgegeben. Da nicht das gesamte blaue Licht umgewandelt wird, ergibt die resultierende additive Mischung der Spektralfarben (siehe Ergebnisspektrum gemäß glatter Linie) ein polychromatisches Licht mit einem ersten engbandigen spektralen Maximum im Spektralbereich A von ca. 460 nm und einem zweiten breitbandigen spektralen Maximum im Spektralbereich B von 510 - 590 nm. Diese beiden Spektralbereiche sind durch ein spektrales Minimum in Bereich von ca. 485 nm getrennt. Hierbei ist es insbesondere vorteilhaft, dass durch die engbandige Emissionsstrahlung des Primäremitters ein kurzwelliger Messwellenbereich zur Bestimmung des Bilirubins anhand dessen Absorptionsmaximums bei ca. 460 nm vorliegt und durch die breitbandige Emissionsstrahlung der Sekundäremitter ein breiter langwelligerer Bereich vorliegt, in welchem alle zu bestimmenden Hämoglobinderivate signifikant absorbieren und Absorptionsmaxima aufweisen.

Das Spektrum derartiger polychromatischer Lumineszenzkonversions-LEDs kann somit erfindungsgemäß über die Wahl des Primäremitters (LED-Chip, welcher im Spektralbereich A emittiert) und die Wahl und Kombination verschiedener Luminophore (Sekundäremitter, welche im Spektralbereich B emittieren) spezifisch auf weitere nachzuweisende Analyten eingestellt werden.

## Patentansprüche

1. Oximeter zur spektralphotometrischen in vitro-Bestimmung von Hämoglobinderivaten in einer Probe mit einer einzigen Messlichtquelle (1), welche eine Messstrahlung emittiert, mit einer Probenkammer (5) und einer Detektionseinrichtung (9, 10), **dadurch gekennzeichnet, dass** die Messlichtquelle (1) eine polychromatische LED ist, welche zur Bestimmung der Hämoglobinderivate Messstrahlung zumindest in einem Spektralbereich B emittiert, in welchem die Hämoglobinderivate eine signifikante Absorption aufweisen und zur Erfassung mindestens eines weiteren Analyten Messstrahlung zumindest in einem weiteren Spektralbereich A emittiert, in welchem der mindestens eine weitere Analyt eine signifikante Absorption aufweist.

2. Oximeter nach Anspruch 1, **dadurch gekennzeichnet, dass** die Messlichtquelle (1) eine Lumineszenzkonversions-LED ist, welche mindestens einen Primäremitter und mindestens einen Sekundäremitter beinhaltet, wobei der Primäremitter Messstrahlung im Spektralbereich A und der Sekundäremitter im Spektralbereich B emittiert.

3. Oximeter nach Anspruch 1, **dadurch gekennzeichnet, dass** die Messlichtquelle (1) mehrere lichtemittierende Schichten mit unterschiedlichen Emissionsspektren beinhaltet, wobei zumindest eine der lichtemittierenden Schichten Messstrahlung im Spektralbereich A und zumindest eine weitere der lichtemittierenden Schichten Messstrahlung in einem Spektralbereich B emittiert, und wobei die lichtemittierenden Schichten innerhalb der Messlichtquelle (1) so zueinander angeordnet sind, dass die Messstrahlung über die zur Messung genutzte Austrittsfläche der Messlichtquelle (1) bzw. über den zur Messung genutzten Abstrahlwinkelbereich der Messlichtquelle (1) ein im Wesentlichen homogenes Spektrum aufweist.

4. Oximeter nach Anspruch 3, **dadurch gekennzeichnet, dass** die Messlichtquelle (1) mehrere lichtemittierende Schichten mit unterschiedlichen Emissionsspektren beinhaltet, welche Schichten zumindest teilweise transparent sind und stapelartig übereinander angeordnet sind.

5. Oximeter nach einem der vorherigen Ansprüche, **dadurch gekennzeichnet, dass** der weitere Analyt Bilirubin ist und der Spektralbereich A der Messstrahlung im Bereich von 450 - 500 nm liegt.

6. Oximeter nach einem der vorherigen Ansprüche, **dadurch gekennzeichnet, dass** der Spektralbereich B der Messstrahlung im Bereich von 520 - 670 nm liegt.

7. Oximeter nach einem der vorherigen Ansprüche, **dadurch gekennzeichnet, dass** der Spektralbereich A und Spektralbereich B der Messstrahlung durch einen Bereich geringerer Intensität getrennt sind.

8. Oximeter nach einem der vorherigen Ansprüche, **dadurch gekennzeichnet, dass** weitere optische Komponenten (3, 4, 7, 8) für die Weiterleitung der Messstrahlung von der Messlichtquelle (1) zur Probenkammer (5) und/oder von der Probenkammer (5) zur Detektionseinrichtung (9, 10) vorhanden sind.

9. Oximeter nach einem der vorherigen Ansprüche, **dadurch gekennzeichnet, dass** zur weiteren spektralen Anpassung der Messstrahlung Filterelemente (7) zwischen der Messlichtquelle (1) und der Probenkammer (5) und/oder zwischen der Probenkammer (5) und der Detektionseinrichtung (9, 10) vorhanden sind.

10. Oximeter nach einem der vorherigen Ansprüche, **dadurch gekennzeichnet, dass** die Detektionseinrichtung aus einem Polychromator (9) und einer nachgeschalteten Multi-Channel-Detektionseinheit (10), beispielsweise einem alle Messwellenlängen simultan erfassenden Detektorarray, besteht.

11. Oximeter zur spektralphotometrischen in vitro-Bestimmung von Hämoglobinderivaten in einer Probe mit einer Messlichtquelle (1), welche eine Messstrahlung emittiert, mit einer Probenkammer (5) und einer Detektionseinrichtung (9, 10), **dadurch gekennzeichnet, dass** die Messlichtquelle eine polychromatische LED, insbesondere eine Weißlicht-LED, ist.
